# EUROPEAN PATENT SPECIFICATION

(11) **EP 4 115 829 B1**
(45) Date of publication and mention of the grant of the patent: **26.03.2025**
(21) Application number: 20956386.5
(22) Date of filing: 08.10.2020
(51) Int. Cl.: A61B 18/14, A61N 1/36, A61N 1/05, A61B 18/00

(54) **ELECTRODE APPARATUS FOR BLOCKING OR CONTROLLING NERVES IN BODY**
ELEKTRODENVORRICHTUNG ZUR BLOCKIERUNG ODER KONTROLLE VON NERVEN IM KÖRPER
APPAREIL À ÉLECTRODES POUR BLOQUER OU COMMANDER DES NERFS DANS UN CORPS

(30) Priority: 29.09.2020 KR 20200126817
(43) Date of publication of application: 11.01.2023
(73) Proprietor: Deepqure Inc., Seoul 03136 (KR)
(72) Inventor: JEONG, Chang Wook, Seoul 05812 (KR); CHANG, Seok Joo, Seoul 08809 (KR); BACH, Du Jin, Seongnam-si Gyeonggi-do 13506 (KR); JO, Seok Hyeon, Namyangju-si Gyeonggi-do 12167 (KR)
(74) Representative: Manitz Finsterwald Patent- und Rechtsanwaltspartnerschaft mbB
(86) International application number: PCT/KR2020/013772
(87) International publication number: WO 2022/071621

(56) References cited:
- WO-A1-2019/118725
- KR-A- 20160 007 087
- KR-A- 20160 088 393
- KR-A- 20190 074 291
- KR-A- 20190 102 486
- US-A1- 2017 056 028
- US-A1- 2019 133 681

## Description

### TECHNICAL FIELD

The present disclosure relates to an electrode apparatus for nerve denervation or modulation in body.

### BACKGROUND

A denervation is a surgical procedure intended to control an abnormally overactive autonomic nervous system by damaging specific nerves. For example, a renal denervation can treat hypertension and heart diseases by damaging renal sympathetic nerves directed to the kidney, and a pulmonary denervation can treat lung diseases by damaging parasympathetic nerves directed to the lung.

Nerves usually enclose the outer walls of tubes, such as blood vessels, bronchial tubes, etc., and it may be necessary to enclose the outer walls of tubes to measure signals from the nerves or transmit electrical impulses or various energies to the nerves to damage or destroy the nerves. For example, when a surgical procedure is performed on the renal artery, the main renal artery which is a procedure target has a diameter of from 5 mm to 7 mm, and the accessory renal artery having a diameter of from 1 mm to 2 mm may also be a procedure target. Also, the artery with distributed nerves varies in size from person to person and has different sizes depending on the location.

When the surgical procedure is performed as described above, it is important to delicately locate a component including an electrode to be formed at the end of a catheter so as to enclose the outer wall of the artery. Specifically, in order to effectively denervate or modulate the nerves, the component needs to enclose the outer wall of the artery with distributed nerves in a circumferential direction. Also, it is necessary to reliably and rapidly enclose the artery with the component including the electrode.

Systems for perivascular nerve denervaion are known from US 2019/133681.

### DISCLOSURE OF THE INVENTION

### PROBLEMS TO BE SOLVED BY THE INVENTION

The present disclosure is conceived to provide an electrode device having a component that guides an electrode to be disposed around a tube in the body as a plurality of unit elements are connected to each other and deformed.

Also, the present disclosure is conceived to provide an electrode device having a configuration in which a plurality of unit elements are connected and deformed so that an electrode completely surrounds the circumference of a tube in the body.

Further, the present disclosure is conceived to provide an electrode apparatus in which a component connected to a plurality of unit elements and configured to guide an electrode is manufactured as a single member without assembly.

The problems to be solved by the present disclosure are not limited to the above-described problems. There may be other problems to be solved by the present disclosure.

### MEANS FOR SOLVING THE PROBLEMS

According to an aspect of the present disclosure, An electrode apparatus for nerve denervation or modulation in body includes a main body including a shaft; an electrode unit formed to be drawn out from one end of the shaft and configured to denervate or modulate at least part of nerves on a tube in the body; and an electrode guide coupled to the electrode unit and deformed into a wound state to bring the electrode unit into contact with the tube in the body. The electrode guide includes a plurality of joint units disposed to enclose the circumference of the tube with the electrode unit interposed therebetween in the wound state.

According to the present disclosure, each joint unit includes a hinge unit formed on one or both sides of the joint unit in a longitudinal direction to be connected to an adjacent joint unit; and a winding support unit formed on one or both sides of the joint unit in the longitudinal direction to support the adjacent joint unit in the wound state.

According to the present disclosure, the electrode guide further includes a tip joint coupled to the electrode unit and connected to the shaft by the plurality of joint units, and in the wound state, a radius of curvature formed by a plurality of joint units located close to the tip joint is smaller than a radius of curvature formed by a plurality of joint units located close to the shaft.

According to the present disclosure, the plurality of joint units is made of an elastically deformable material and formed as one body, and a winding support groove of which at least a part of a space is deformed to be closed in the wound state is formed between adjacent joint units of the electrode guide.

The above-described aspects are provided by way of illustration only and should not be construed as liming the present disclosure. Besides the above-described embodiments, there may be additional embodiments described in the accompanying drawings and the detailed description.

### EFFECTS OF THE INVENTION

According to an electrode apparatus of the present disclosure, joint units are driven to deform an electrode guide into a wound state in order to bring an electrode into close contact with an outer surface of a tube and efficiently transfer energy. Therefore, it is possible to reliably control the location of an electrode unit.

Further, according to the electrode apparatus of the present disclosure, joint units are formed to have different lengths or shapes, and, thus, the shape of the electrode guide in the wound state can be precisely designed and the electrode guide can be located to completely enclose the tube in the body. Accordingly, a surgical procedure for denervating or modulating nerves can be effectively performed.

Meanwhile, according to the electrode apparatus of the present disclosure, the electrode guide including the plurality of joint units as one body is formed while implementing driving of the plurality of joint units. Thus, the electrode apparatus can be manufactured through a simple process and produced in a small size, which results in a reduction in manufacturing cost.

### BRIEF DESCRIPTION OF THE DRAWINGS

**FIG.** 1 is a side view of an electrode apparatus according to an embodiment of the present disclosure.
**FIG. 2** is a perspective view illustrating a wound state of an electrode guide illustrated in **FIG. 1****.**
**FIG. 3A** through **FIG. 3C** illustrate a process of deforming the electrode guide into a wound state according to an embodiment of the present disclosure.
**FIG. 4** is a perspective view illustrating joint units and a tip joint illustrated in **FIG. 2****.**
**FIG.** 5 is an exploded perspective view illustrating a portion of the joint units illustrated in **FIG. 4****.**
**FIG.** 6 is a side view illustrating a portion of the joint units illustrated in **FIG. 4****.**
**FIG.** 7 is a perspective view of an electrode guide according to another embodiment of the present disclosure.

### BEST MODE FOR CARRYING OUT THE INVENTION

Hereafter, example embodiments will be described in detail with reference to the accompanying drawings so that the present disclosure may be readily implemented by those skilled in the art. However, it is to be noted that the present disclosure is not limited to the example embodiments but can be embodied in various other ways. In the drawings, parts irrelevant to the description are omitted for the simplicity of explanation, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element. Further, it is to be understood that the term "comprises or includes" and/or "comprising or including" used in the document means that one or more other components, steps, operation and/or existence or addition of elements are not excluded in addition to the described components, steps, operation and/or elements unless context dictates otherwise and is not intended to preclude the possibility that one or more other features, numbers, steps, operations, components, parts, or combinations thereof may exist or may be added. Through the whole document, the term "on" that is used to designate a position of one element with respect to another element includes both a case that the one element is adjacent to the other element and a case that any other element exists between these two elements.

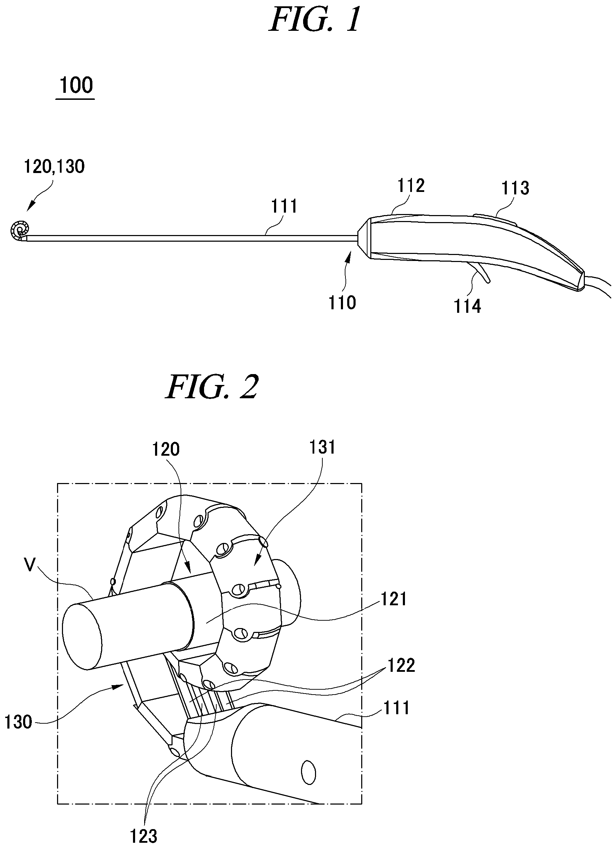

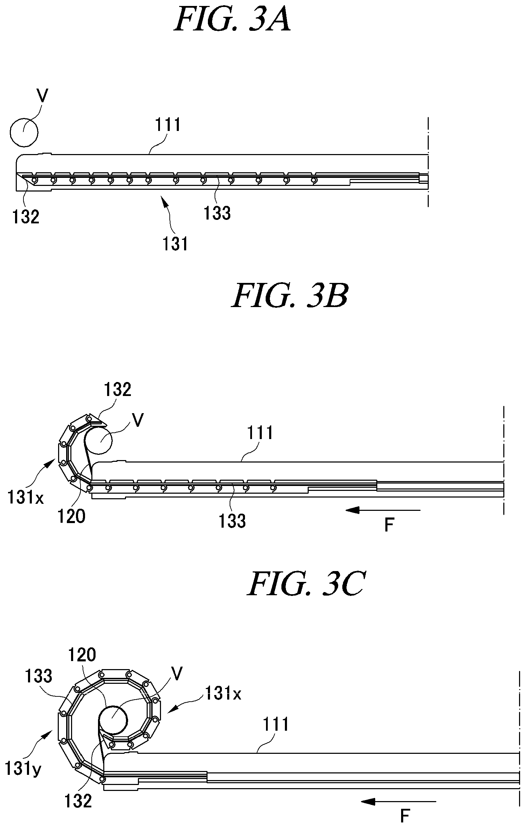

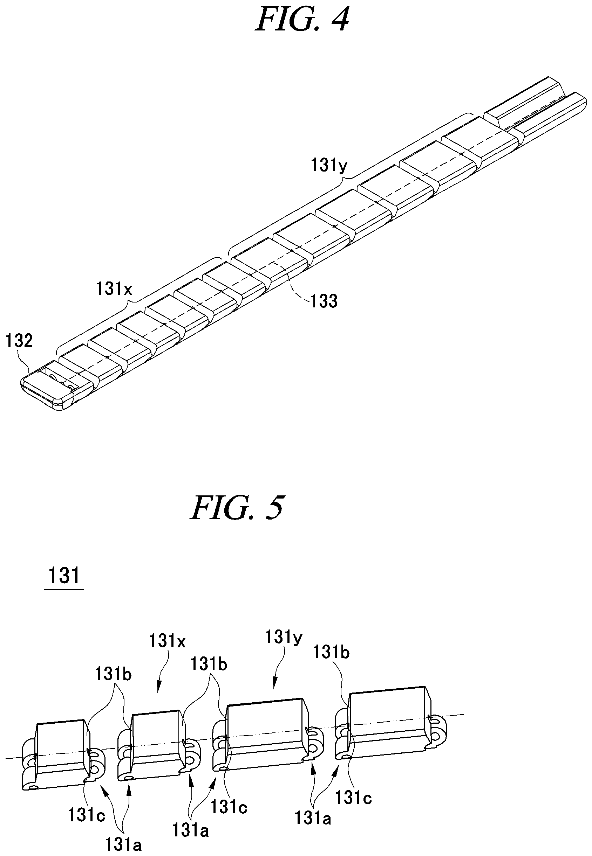

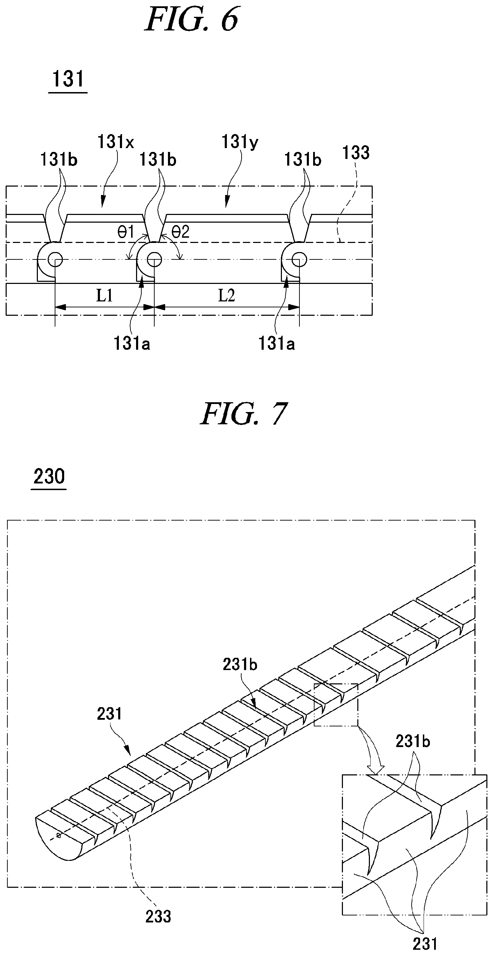

**FIG.** 1 is a side view of an electrode apparatus according to an embodiment of the present disclosure and **FIG.** 2 is a perspective view illustrating a wound state of an electrode guide illustrated in **FIG. 1****.** **FIG. 3A** through **FIG. 3C** illustrate a process of deforming the electrode guide into a wound state according to an embodiment of the present disclosure. Further, **FIG.** 4 is a perspective view illustrating joint units and a tip joint illustrated in **FIG. 2****,** **FIG. 5** is an exploded perspective view illustrating a portion of the joint units illustrated in **FIG. 4** **and** **FIG. 6** is a side view illustrating a portion of the joint units illustrated in **FIG. 4****.**

Referring to **FIG. 1****,** an electrode apparatus 100 according to an embodiment of the present disclosure includes a main body 110, an electrode unit 120 and an electrode guide 130.

The main body 110 may include the shaft 111 extending in one direction, a grip portion 112 connected to the shaft 111 so as to be gripped by an operator, a guide manipulation unit 113 formed on the grip portion 112 so as to manipulate an operation of the electrode guide 130, and an electrode manipulation unit 114 formed on the grip portion 112 so as to manipulate an operation of the electrode unit 120. The components for driving and controlling the electrode unit 120 and the electrode guide 130 may be located inside the main body 110.

The electrode unit 120 is formed to be drawn out from one end of the shaft 111 and configured to denervate or modulate at least part of nerves distributed on a tissue in the body including a tube depending on manipulation by the operator.

Referring to **FIG. 2****,** the electrode unit 120 may include a substrate portion 121, an electrode unit 122 and a sensor unit 123. In the electrode apparatus 100 according to the present disclosure, an electrode encloses an outer surface of a tube or tube-shaped tissue V in the body and energy can be transferred through the electrode. To this end, the substrate portion 121 may be formed as a flexible printed circuit board (FPCB).

The electrode unit 122 is formed on the substrate portion 121, and in the embodiment illustrated in **FIG.** 2, the electrode unit 122 may be composed of two electrodes extending parallel to each other on the substrate portion 121. In the present embodiment, the substrate portion 121 and the electrode unit 122 may be configured to extend in a circumferential direction and enclose the tube in the body or the like.

The electrode unit 122 may be made of a material such as stainless steel or gold, which is harmless to the human body and conducts electricity well, in order to block or denervate or control or modulate the nerves. Also, the electrode unit 122 may transfer various types of energy from an energy source generator. For example, the energy may include radio-frequency (RF) energy, electrical energy, laser energy, ultrasonic energy, high-intensity focused ultrasound energy, cryogenic energy and other heat energy.

Also, the electrode unit 122 may be implemented as a flexible PCB for transferring RF energy, a transducer for transferring ultrasonic energy or a metal electrode for transferring high-voltage energy and thus may transfer energy to damage the nerves.

Further, the sensor unit 123 may be formed on the substrate portion 121. For example, the sensor unit 123 may be a thermocouple that measures a temperature by contacting with the tube in the body or the like, and when neurotomy is performed with the electrode apparatus 100 according to the present disclosure, the sensor unit 123 may monitor a temperature of a treatment site. As another example, the sensor unit 123 may measure signals from the nerves on the tube.

The electrode guide 130 functions to bring the electrode unit 120 into contact with the tube in the body. The electrode guide 130 is coupled to the electrode unit 120 and deformed into a wound state to bring the electrode unit 120 into contact with the tube in the body.

Referring to **FIG. 3A** through **FIG. 3C** and **FIG. 4****,** the electrode guide 130 of the present disclosure includes a plurality of joint units 131 in order to be deformed into the wound state. In the wound state, the plurality of joint units 131 is disposed to enclose the circumference of the tube V in the body with the electrode unit 120 interposed therebetween. For example, the state illustrated in **FIG. 2** and **FIG. 3C** may be the wound state.

According to the electrode apparatus 100 of the present disclosure, the joint units 131 may be driven to deform the electrode guide 130 into the wound state in order to bring the electrode unit 122 into close contact with the outer surface of the tube in the body and efficiently transfer energy. Such a joint driving mechanism makes it possible to control an operation timing and the shape of the electrode guide 130 directly and improve the reliability of repetitive operation, as compared to a conventional shape memory material mechanism. Therefore, it is possible to perform a customized and detailed surgical procedure using the electrode apparatus 100 of the present disclosure.

According to an embodiment of the present disclosure, the electrode guide 130 is accommodated together with the electrode unit 120 inside the shaft 111 and may protrude from one end in a forward direction F while being deformed into the wound state at the time of surgical procedure. As illustrated in **FIG. 3A** through **FIG.** 3C, when the plurality of joint units 131 is sequentially drawn out, the plurality of joint units 131 may move toward one direction and thus may overall enclose the tube V in the wound state. In the wound state, the electrode guide 130 is spaced apart from an outer circumferential surface of the tube and the electrode unit 120 located inside the wound electrode guide 130 may be in close contact with the outer circumferential surface of the tube V.

Hereafter, the detailed configuration of the electrode unit 130 and the joint units 131 will be described with further reference to **FIG. 5** and **FIG. 6****.**

The electrode guide 130 may further include a tip joint 132 and a wire 133. The tip joint 132 may be connected to the shaft 111 by the plurality of joint units 131 and coupled to the electrode unit 120. As illustrated in **FIG.** 3C, the tip joint 132 may be located close to the tube V in the body in the wound state and may have a shape that gradually decreases in width or thickness toward the end in order to suppress interference with the electrode unit 120 or maximize the surface enclosing the tube in the body. The end of the electrode unit 120 may be fastened and fixed to the tip joint 132.

The wire 133 may be formed to sequentially penetrate the plurality of joint units 131. Referring to **FIG.** 5, each joint unit 131 may have a through-hole 131c in a longitudinal direction to allow penetration of the wire 133. The end of the wire 133 sequentially penetrating the through-holes 131c may be coupled and fixed to the tip joint 132, and the wire 133 can slide with respect to each joint unit 131 in the through-hole 131c in the longitudinal direction. Therefore, the wire 133 can guide the plurality of joint units 131 and the tip joint 132 to be in the wound state and support the tip joint 132 and the plurality of joint units 131 in the wound state.

Meanwhile, each join unit 131 may include hinge units 131a and winding support units 131b. The hinge units 131a are configured for rotatable connection to adjacent joints and may be formed on one or both sides of the joint unit 131 in the longitudinal direction in which the joint units 131 are connected parallel to each other. As illustrated in **FIG.** 5, the hinge unit 131a may have a rotation axis in a direction intersecting the longitudinal direction so as to be connected to the hinge unit 131a of the adjacent joint unit 131. A hinge pin (not illustrated) may be inserted into and fastened to each hinge unit 131a in the direction of the rotation axis.

The winding support units 131b are configured to maintain the wound state and may be formed on one or both sides of the joint unit 131 in the longitudinal direction to support the adjacent joint unit 131. As illustrated in **FIG.** 5, the winding support unit 131b may be located adjacent to the hinge unit 131a in an inward direction of the electrode guide 130 (in a direction of winding the joint unit 131). For example, the winding support unit 131b may be formed as a surface having a predetermined angle and area and supported by the adjacent winding support unit 131b in surface contact with each other in the wound state, and, thus, a wound shape of the electrode guide 130 can be maintained.

In the embodiment illustrated in **FIG. 3A** and **FIG. 3C****,** when the wire 133 is pulled backwards relative to the electrode guide 130 (when a length of the wire 133 drawn out from the shaft 111 is smaller than that of the electrode guide 130), a tensile force may be applied to the wire 133 in a direction of winding the electrode guide 130. On the other hand, the winding support units 131b may provide a force of supporting the joint units 131 to each other in a direction of suppressing winding of the electrode guide 130. That is, since the wire 133 and the winding support units 131b form a balanced force in opposite directions, the electrode guide 130 can be fixed in the wound state.

More specifically, the through-hole 131c may be formed at a location spaced apart from a rotation center of the hinge unit 131a in an inward direction (in an upward direction in **FIG. 6**). When the wire 133 of which the end is fixed to the tip joint 132 is pulled relatively backwards (toward a right direction in **FIG. 6**), the plurality of joint units 131 may be bent overall in an inward direction. When the joint unit 131 is rotated around the hinge unit 131a with respect to the adjacent joint unit 131 and the winding support units 131b are in contact with each other, the locations of the plurality of joint units 131 may be fixed and the plurality of joint units 131 may be in the wound state. Here, the wire 133 may provide a force (tensile force) of supporting the winding support units 131b to each other.

As described above, a change in location in the wound state is suppressed by the wire 133 and the winding support units 131b, and, thus, the electrode guide 130 of the electrode apparatus 100 according to the present disclosure can maintain its location during a surgical procedure.

Hereafter, an embodiment where the shape of the electrode guide 130 can be set in the wound state due to a difference in shape between the joint units 131 will be described.

According to an embodiment of the present disclosure, the electrode guide 130 may include a first joint group 131x and a second joint group 131y. That is, the plurality of joint units 131 may be divided into the first joint group 131x and the second joint group 131y having different shapes.

In the embodiment illustrated in **FIG. 2** through **FIG. 6**, the first joint group 131x may include joint units 131 each having a first length L1 in the longitudinal direction, and the second joint group 131y may include joint units 131 each having a second length L2 greater than the first length L1. In the present embodiment, each of the first joint group 131x and the second joint group 131y may include, for example, six joint units 131 each having the same length.

Due to such a difference in length, the first joint group 131x may form a first radius of curvature and the second joint group 131y may form a second radius of curvature greater than the first radius of curvature in the wound state. As can be seen from **FIG. 3C**, the joint units (the first joint group 131x) having a relatively small length may form a smaller radius of curvature and the joint units (the second joint group 131y) having a relatively great length may form a greater radius of curvature.

More specifically, the first joint group 131x forming the first radius of curvature may be located close to the tip joint 132, and the second joint group 131y forming the second radius of curvature may be located close to the shaft 111.

When the joint units 131 located close to the tip joint 132 form a smaller radius of curvature in the wound state, a path along which the tip joint 132 enters a space between the tube in the body and the shaft 111 may be formed as shown in **FIG. 3C****.** For example, the electrode guide 130 including the joint units 131 may have an overall spiral shape.

As described above, in the electrode apparatus 100 according to the present disclosure, the shape of the electrode guide 130 in the wound state can be easily and precisely set by designing the lengths of the plurality of joint units 131. Also, it is possible to secure excellent repeatability in the shape in the wound state. Further, the electrode guide 130 can be located to fully enclose the tube in the body in the wound state by varying the radius of curvature. Therefore, it is possible to generally denervate or modulate the nerves around the tube in a one-time surgical procedure and thus possible to increase the treatment effect.

In the embodiment illustrated in **FIG. 2** through **FIG. 6****,** it is assumed that all of the joint units 131 have a uniform tilt angle with respect to the adjacent joint units 131. Specifically, each joint unit 131 may be disposed in the longitudinal direction to intersect the adjacent joint unit 131 at a tilt angle of, for example, 30°. To this end, the respective surfaces of the winding support units 131b of the joint units 131 may have tilt angles θ1 and θ2 of, for example, 75° with respect to the longitudinal direction.

In another embodiment of the electrode guide 130 of the present disclosure, the joint units 131 having the same length may have a plurality of radiuses of curvature in the wound state. As described above, a tilt angle between the adjacent joint units 131 in the wound state may be determined by tilt angles of the surfaces of the winding support units 131b with respect to the longitudinal direction of the joint units 131 when the joint units 131 are designed.

Specifically, a first joint group may include joint units in which surfaces of winding support units have a first angle with respect to the longitudinal direction, and a second joint group may include joint units in which surfaces of winding support units have a second angle greater than the first angle. Thus, when the first joint group having the first angle has a first radius of curvature in the wound state, the second joint group having the second angle may be disposed to have a second radius of curvature greater than the first radius of curvature.

Therefore, even if all of the joint units have the same length, it is possible to implement a wound state where the joint units have different radiuses of curvature by designing the winding support units differently from each other.

Although the embodiment where the joint units 131 include two joint groups has been described above, it is also possible to more delicately design a winding path by designing the electrode guide 130 to include two or more joint groups having different shapes.

**FIG.** 7 is a perspective view of an electrode guide 230 according to another embodiment of the present disclosure. Hereafter, an embodiment where joint units 231 of the electrode guide 230 of the present disclosure are formed as one body will be described.

The joint units 231 of the electrode guide 230 according to another embodiment of the present disclosure may be made of a material such as elastically deformable polymer, and a plurality of joint units 231 may be formed as one body, for example, a living hinge structure.

As illustrated in **FIG. 7****,** each joint unit 231 may be formed as one body with another joint unit 231 adjacent to each other in the longitudinal direction, and a winding support groove 231b may be formed between the adjacent joint units 231. At least a part of a space in the winding support groove 231b may be reduced or closed in the wound state.

Specifically, the winding support groove 231b may be formed to be recessed in a wedge shape in the electrode guide 230's inner surface (a surface facing the electrode unit 120). In the wound state, side surfaces of the wedge-shaped winding support grooves 231b may be in contact with each other and may be supported by each other.

The electrode guide 230 according to another embodiment of the present disclosure may further include a wire 233. The wire 233 may be formed to sequentially penetrate the plurality of joint units 231. As in the above-described embodiment, a length of the wire 233 drawn out from the shaft 111 is smaller than that of the electrode guide 230, and, thus, the wire 233 can guide the electrode guide 230 to be deformed into a shape enclosing the tube and provide a force of closing and supporting at least part of the winding support grooves 231b.

The electrode guide 230 according to another embodiment of the present disclosure can be manufactured as one body while implementing a reliable operation of the joint units. Since it is not necessary to assemble separately manufactured joint elements, the electrode guide 230 can be manufactured through a simple process and produced in a small size, which results in a reduction in manufacturing cost.

The above description of the present disclosure is provided for the purpose of illustration, and it would be understood by a person with ordinary skill in the art that various changes and modifications may be made without changing technical conception and essential features of the present disclosure. Thus, it is clear that the above-described embodiments are illustrative in all aspects and do not limit the present disclosure.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. An electrode apparatus for nerve denervation or modulation in body, comprising:
a main body including a shaft;
an electrode unit formed to be drawn out from one end of the shaft and configured to denervate or modulate at least part of nerves on a tube in the body; and
an electrode guide coupled to the electrode unit and deformed into a wound state to bring the electrode unit into contact with the tube in the body;
wherein the electrode guide includes a plurality of joint units disposed to enclose the circumference of the tube with the electrode unit interposed therebetween in the wound state.

2. The electrode apparatus of Claim 1,
wherein each joint unit includes:
a hinge unit formed on one or both sides of the joint unit in a longitudinal direction to be connected to an adjacent joint unit; and
a winding support unit formed on one or both sides of the joint unit in the longitudinal direction to support the adjacent joint unit in the wound state.

3. The electrode apparatus of Claim 1,
wherein in the wound state, the electrode guide includes:
a first joint group including a plurality of joint units each having a first length in a longitudinal direction to be connected to an adjacent joint unit and disposed to form a first radius of curvature; and
a second joint group including a plurality of joint units each having a second length greater than the length of each joint unit of the first joint group in the longitudinal direction and disposed to form a second radius of curvature greater than the first radius of curvature.

4. The electrode apparatus of Claim 2,
wherein in the wound state, the joint units include:
a first joint group including a plurality of joint units in which surfaces of the winding support units have a first angle with respect to the longitudinal direction and which is disposed to form a first radius of curvature; and
a second joint group including a plurality of joint units in which surfaces of the winding support units have a second angle greater than the first angle with respect to the longitudinal direction and which is disposed to form a second radius of curvature greater than the first radius of curvature.

5. The electrode apparatus of Claim 1,
wherein the electrode guide further includes a tip joint coupled to the electrode unit and connected to the shaft by the plurality of joint units.

6. The electrode apparatus of Claim 1,
wherein the electrode guide further includes a wire formed to sequentially penetrate the plurality of joint units and configured to guide the plurality of joint units to be in the wound state.

7. The electrode apparatus of Claim 5,
wherein the electrode guide further includes a wire formed to sequentially penetrate the plurality of joint units and coupled to the tip joint and configured to guide the location of the tip joint in the wound state.

8. The electrode apparatus of Claim 2,
wherein each joint unit further includes:
a through-hole formed in the longitudinal direction at a location spaced apart from a rotation center of the hinge unit, and
the electrode guide further includes a wire inserted into the through-hole and configured to provide a force of supporting the winding support units to each other between adjacent joint units.

9. The electrode apparatus of Claim 5,
wherein in the wound state, a radius of curvature formed by a plurality of joint units located close to the tip joint is smaller than a radius of curvature formed by a plurality of joint units located close to the shaft.

10. The electrode apparatus of Claim 1,
wherein the plurality of joint units is made of an elastically deformable material and formed as one body, and
a winding support groove of which at least a part of a space is deformed to be closed in the wound state is formed between adjacent joint units of the electrode guide.

11. The electrode apparatus of Claim 10,
wherein the winding support groove is recessed in a wedge shape in the electrode guide's surface facing the electrode unit.

12. The electrode apparatus of Claim 10,
wherein the electrode guide further includes a wire formed to sequentially penetrate the plurality of joint units and provides a force of closing and supporting at least part of the winding support grooves in the wound state.

## Patentansprüche

1. Elektrodenvorrichtung zur Denervierung oder Modulation von Nerven im Körper, umfassend:
einen Hauptkörper mit einem Schaft;
eine Elektrodeneinheit, die ausgebildet ist, um aus einem Ende des Schafts herausgezogen zu werden, und konfiguriert ist, um mindestens einen Teil von Nerven an einer Röhre im Körper zu denervieren oder zu modulieren;
und
eine Elektrodenführung, die mit der Elektrodeneinheit gekoppelt und in einen gewickelten Zustand verformt ist, um die Elektrodeneinheit mit der Röhre im Körper in Kontakt zu bringen;
wobei die Elektrodenführung eine Vielzahl von Gelenkeinheiten umfasst, die angeordnet sind, um den Umfang der Röhre zu umschließen, wobei die Elektrodeneinheit im gewickelten Zustand dazwischen angeordnet ist.

2. Elektrodenvorrichtung nach Anspruch 1,
wobei jede Gelenkeinheit umfasst:
eine Scharniereinheit, die auf einer oder beiden Seiten der Gelenkeinheit in einer Längsrichtung ausgebildet ist, um mit einer benachbarten Gelenkeinheit verbunden zu werden; und
eine Wickelstützeinheit, die auf einer oder beiden Seiten der Gelenkeinheit in der Längsrichtung ausgebildet ist, um die benachbarte Gelenkeinheit im gewickelten Zustand zu stützen.

3. Elektrodenvorrichtung nach Anspruch 1,
wobei die Elektrodenführung im gewickelten Zustand umfasst:
eine erste Gelenkgruppe mit einer Vielzahl von Gelenkeinheiten, die jeweils eine erste Länge in einer Längsrichtung aufweisen, um mit einer benachbarten Gelenkeinheit verbunden zu werden, und angeordnet sind, um einen ersten Krümmungsradius zu bilden; und
eine zweite Gelenkgruppe mit einer Vielzahl von Gelenkeinheiten, die jeweils eine zweite Länge aufweisen, die größer als die Länge jeder Gelenkeinheit der ersten Gelenkgruppe in der Längsrichtung ist, und angeordnet sind, um einen zweiten Krümmungsradius zu bilden, der größer als der erste Krümmungsradius ist.

4. Elektrodenvorrichtung nach Anspruch 2,
wobei die Gelenkeinheiten im gewickelten Zustand umfassen:
eine erste Gelenkgruppe mit einer Vielzahl von Gelenkeinheiten, in denen Oberflächen der Wickelstützeinheiten einen ersten Winkel in Bezug auf die Längsrichtung aufweisen und die angeordnet ist, um einen ersten Krümmungsradius zu bilden; und
eine zweite Gelenkgruppe mit einer Vielzahl von Gelenkeinheiten, in denen Oberflächen der Wickelstützeinheiten einen zweiten Winkel aufweisen, der größer als der erste Winkel in Bezug auf die Längsrichtung ist, und die angeordnet ist, um einen zweiten Krümmungsradius zu bilden, der größer als der erste Krümmungsradius ist.

5. Elektrodenvorrichtung nach Anspruch 1,
wobei die Elektrodenführung ferner ein Spitzengelenk umfasst, das mit der Elektrodeneinheit gekoppelt und durch die Vielzahl von Gelenkeinheiten mit dem Schaft verbunden ist.

6. Elektrodenvorrichtung nach Anspruch 1,
wobei die Elektrodenführung ferner einen Draht umfasst, der ausgebildet ist, um nacheinander die Vielzahl von Gelenkeinheiten zu durchdringen, und konfiguriert ist, um die Vielzahl von Gelenkeinheiten zu führen, damit sie sich im gewickelten Zustand befinden.

7. Elektrodenvorrichtung nach Anspruch 5,
wobei die Elektrodenführung ferner einen Draht umfasst, der ausgebildet ist, um nacheinander die Vielzahl von Gelenkeinheiten zu durchdringen, und mit dem Spitzengelenk gekoppelt und konfiguriert ist, um die Position des Spitzengelenks im gewickelten Zustand zu führen.

8. Elektrodenvorrichtung nach Anspruch 2,
wobei jede Gelenkeinheit ferner umfasst:
ein Durchgangsloch, das in der Längsrichtung an einer Position ausgebildet ist, die von einem Drehzentrum der Scharniereinheit beabstandet ist, und
die Elektrodenführung ferner einen Draht umfasst, der in das Durchgangsloch eingeführt und konfiguriert ist, um eine Kraft zum Stützen der Wickelstützeinheiten aneinander zwischen benachbarten Gelenkeinheiten bereitzustellen.

9. Elektrodenvorrichtung nach Anspruch 5,
wobei im gewickelten Zustand ein Krümmungsradius, der durch eine Vielzahl von Gelenkeinheiten gebildet wird, die sich nahe dem Spitzengelenk befinden, kleiner ist als ein Krümmungsradius, der durch eine Vielzahl von Gelenkeinheiten gebildet wird, die sich nahe dem Schaft befinden.

10. Elektrodenvorrichtung nach Anspruch 1,
wobei die Vielzahl von Gelenkeinheiten aus einem elastisch verformbaren Material besteht und als ein Körper ausgebildet ist, und
eine Wickelstütznut, von der mindestens ein Teil eines Raums verformt ist, um im gewickelten Zustand geschlossen zu werden, zwischen benachbarten Gelenkeinheiten der Elektrodenführung ausgebildet ist.

11. Elektrodenvorrichtung nach Anspruch 10,
wobei die Wickelstütznut in einer Keilform in der Oberfläche der Elektrodenführung, die der Elektrodeneinheit zugewandt ist, ausgenommen ist.

12. Elektrodenvorrichtung nach Anspruch 10,
wobei die Elektrodenführung ferner einen Draht umfasst, der ausgebildet ist, um nacheinander die Vielzahl von Gelenkeinheiten zu durchdringen, und eine Kraft zum Schließen und Stützen mindestens eines Teils der Wickelstütznuten im gewickelten Zustand bereitstellt.

## Revendications

1. Appareil à électrode pour la dénervation ou la modulation de nerfs dans le corps, comprenant :
un corps principal incluant une tige ;
une unité d'électrode formée de manière à être extraite d'une extrémité de la tige et configurée pour dénerver ou moduler au moins une partie de nerfs sur un tube dans le corps ; et
un guide d'électrode couplé à l'unité d'électrode et déformé dans un état enroulé pour amener l'unité d'électrode en contact avec le tube dans le corps ;
dans lequel le guide d'électrode comprend une pluralité d'unités de joint disposées de manière à entourer la circonférence du tube avec l'unité d'électrode interposée entre elles dans l'état enroulé.

2. Appareil à électrode selon la revendication 1,
dans lequel chaque unité de joint inclut :
une unité de charnière formée sur un ou les deux côtés de l'unité de joint dans une direction longitudinale pour être reliée à une unité de joint adjacente ; et
une unité de soutien d'enroulement formée sur un ou les deux côtés de l'unité de joint dans la direction longitudinale pour soutenir l'unité de joint adjacente dans l'état enroulé.

3. Appareil à électrode selon la revendication 1,
dans lequel, dans l'état enroulé, le guide d'électrode inclut :
un premier groupe de joints incluant une pluralité d'unités de joint ayant chacune une première longueur dans une direction longitudinale pour être reliée à une unité de joint adjacente et disposé de manière à former un premier rayon de courbure ; et
un deuxième groupe de joints incluant une pluralité d'unités de joint ayant chacune une deuxième longueur plus grande que la longueur de chaque unité de joint du premier groupe de joints dans la direction longitudinale et disposé de manière à former un deuxième rayon de courbure plus grand que le premier rayon de courbure.

4. Appareil à électrode selon la revendication 2,
dans lequel, dans l'état enroulé, les unités de joint incluent :
un premier groupe de joints incluant une pluralité d'unités de joint dans lesquelles les surfaces des unités de soutien d'enroulement font un premier angle par rapport à la direction longitudinale et qui est disposé de manière à former un premier rayon de courbure ; et
un deuxième groupe de joints incluant une pluralité d'unités de joint dans lesquelles les surfaces des unités de soutien d'enroulement font un deuxième angle plus grand que le premier angle par rapport à la direction longitudinale et qui est disposé de manière à former un deuxième rayon de courbure plus grand que le premier rayon de courbure.

5. Appareil à électrode selon la revendication 1,
dans lequel le guide d'électrode inclut en outre un joint d'extrémité couplé à l'unité d'électrode et relié à la tige par la pluralité d'unités de joint.

6. Appareil à électrode selon la revendication 1,
dans lequel le guide d'électrode inclut en outre un fil formé de manière à pénétrer séquentiellement dans la pluralité d'unités de joint et configuré pour guider la pluralité d'unités de joint de manière à les mettre dans l'état enroulé.

7. Appareil à électrode selon la revendication 5,
dans lequel le guide d'électrode inclut en outre un fil formé de manière à pénétrer séquentiellement dans la pluralité d'unités de joint et couplé au joint d'extrémité, configuré pour guider l'emplacement du joint d'extrémité dans l'état enroulé.

8. Appareil à électrode selon la revendication 2,
dans lequel chaque unité de joint inclut en outre :
un trou traversant formé dans la direction longitudinale à un emplacement espacé d'un centre de rotation de l'unité de charnière, et
le guide d'électrode inclut en outre un fil inséré dans le trou traversant et configuré pour fournir une force de soutien des unités de soutien d'enroulement les unes aux autres entre les unités de joint adjacentes.

9. Appareil à électrode selon la revendication 5,
dans lequel, dans l'état enroulé, un rayon de courbure formé par une pluralité d'unités de joint situées à proximité du joint d'extrémité est plus petit qu'un rayon de courbure formé par une pluralité d'unités de joint situées à proximité de la tige.

10. Appareil à électrode selon la revendication 1,
dans lequel la pluralité d'unités de joint est constituée d'un matériau élastiquement déformable et formée comme un seul corps, et
une rainure de soutien d'enroulement dont au moins une partie d'un espace est déformée de manière à être fermée dans l'état enroulé est formée entre les unités de joint adjacentes du guide d'électrode.

11. Appareil à électrode selon la revendication 10,
dans lequel la rainure de soutien d'enroulement est renfoncée en forme de coin dans la surface du guide d'électrode faisant face à l'unité d'électrode.

12. Appareil à électrode selon la revendication 10,
dans lequel le guide d'électrode inclut en outre un fil formé de manière à pénétrer séquentiellement dans la pluralité d'unités de joint et fournit une force de fermeture et de soutien d'au moins une partie des rainures de soutien d'enroulement dans l'état enroulé.
